# EUROPEAN PATENT SPECIFICATION

(11) **EP 2 744 544 B1**
(45) Date of publication and mention of the grant of the patent: **14.12.2016**
(21) Application number: 12787891.6
(22) Date of filing: 05.10.2012
(51) Int. Cl.: A61M 15/00

(54) **MOUTHPIECE FOR DELIVERING NEBULIZED MEDICAMENT TO THE AIRWAY OF A SUBJECT**
MUNDSTÜCK ZUR VERABREICHUNG EINES ZERSTÄUBTEN ARZNEIMITTELS IN DIE ATEMWEGE EINES PATIENTEN
EMBOUT BUCCAL D'ADMINISTRATION D'UN MÉDICAMENT NÉBULISÉ AUX VOIES AÉRIENNES D'UN SUJET

(30) Priority: 10.10.2011 US 201161545315 P
(43) Date of publication of application: 25.06.2014
(73) Proprietor: Koninklijke Philips N.V., 5656 AE Eindhoven (NL)
(72) Inventor: VON HOLLEN, Dirk Ernest, NL-5656 AE Eindhoven (NL)
(74) Representative: Steffen, Thomas
(86) International application number: PCT/IB2012/055385
(87) International publication number: WO 2013/054244

(56) References cited:
- US-A1- 2002 121 275
- US-A1- 2010 319 687
- US-A1- 2011 114 090

## Description

### BACKGROUND

### 1. Field

The present disclosure pertains to a mouthpiece for delivering nebulized medicament to the airway of a subject, and, in particular, to a removable mouthpiece that facilitates operation of a nebulizer in a breath assisted mode that is replaceable with a mouthpiece that facilitates operation of the same nebulizer in continuous use mode.

### 2. Description of the Related Art

The use of nebulizers to nebulize medicament for respiratory delivery to subjects is known. Various types of nebulizers are known. These types include continuous use nebulizers and breath assisted nebulizers. Generally, nebulizers are configured to operate either in continuous mode, or in breath assisted mode.

Continuous use nebulizers typically produce a substantially continuous flow of nebulized medicament for inhalation by a subject. A continuous use nebulizer generally provides a single flow path through which gas is provided to and received from the mouth of the subject. While these nebulizers tend to be efficient at delivering a stream of nebulized medicament, exhalation by the subject back into the nebulizer will usually result in nebulized medicament being exhausted from the nebulizer.

Breath assisted nebulizers are configured such that if a user exhales back into the nebulizer, the exhaled gas is exhausted from the nebulizer without permitting substantial communication between the exhaled gas and nebulized medicament present in the nebulizer and/or the mouthpiece thereof.

US 2002/0121275 A1 discloses a mouthpiece according to the preamble of claim 1.

US 2010/0319687 A1 discloses a nebulizer and inhalation aid used therefor.

### SUMMARY

Accordingly, one or more aspects of the present disclosure relate to a mouthpiece configured to be removably coupled with a nebulizer to deliver nebulized medicament to the airway of a subject. In some embodiments, the mouthpiece comprises one or more of a nebulizer interface, a subject interface, a medicament flow path, an exhaust outlet, an exhaust flow path, an inlet valve, an exhaust valve. The nebulizer interface is configured to releasably engage an outlet of a nebulizer through which the nebulizer outputs nebulized medicament. The subject interface is suitable for engagement by the month of the subject and for to communicating the interior of the mouthpiece with the airway of the subject. The medicament flow path is configured to place the nebulizer interface in fluid communication with the subject interface to facilitate a flow of nebulized medicament from the nebulizer interface to the subject interface and into the airway of the subject through the subject interface. The exhaust flow path is configured to place the subject interface in fluid communication with the exhaust outlet to facilitate a flow of exhaled gas from the subject interface to the exhaust outlet and out of the mouthpiece through the exhaust outlet. The inlet valve is configured to communicate ambient air with the medicament flow path, wherein the inlet valve is a one-way valve that permits flow from ambient atmosphere into the medicament flow path. The exhaust valve controls gas flow within the exhaust flow path, wherein the outlet valve is a one-way valve that permits flow out of the exhaust outlet. The mouthpiece further comprises a dividing structure that physically separates the medicament flow path from the exhaust flow path.

These and other objects, features, and characteristics of the present displosure, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the disclosure.

### BRIEF DESCRIPTION OF THE DRAWINGS

FIG. 1 is an illustration of a nebulizer and mouthpiece;
FIG. 2 is an illustration of a nebulizer and mouthpiece;
FIG. 3 is sectional view of a nebulizer mouthpiece;
FIG. 5 is an illustration of a method of delivering nebulized medicament to the airway of a subject.

### DETAILED DESCRIPTION OF EXEMPLARY EMBODIMENTS

As used herein, the singular form of "a", "an", and "the" include plural references unless the context clearly dictates otherwise. As used herein, the statement that two or more parts or components are "coupled" shall mean that the parts are joined or operate together either directly or indirectly, i.e., through one or more intermediate parts or components, so long as a link occurs. As used herein, "directly coupled" means that two elements are directly in contact with each other. As used herein, "fixedly coupled" or "fixed" means that two components are coupled so as to move as one while maintaining a constant orientation relative to each other.

As used herein, the word "unitary" means a component is created as a single piece or unit. That is, a component that includes pieces that are created separately and then coupled together as a unit is not a "unitary" component or body. As employed herein, the statement that two or more parts or components "engage" one another shall mean that the parts exert a force against one another either directly or through one or more intermediate parts or components. As employed herein, the term "number" shall mean one or an integer greater than one (i.e., a plurality).

Directional phrases used herein, such as, for example and without limitation, top, bottom, left, right, upper, lower, front, back, and derivatives thereof, relate to the orientation of the elements shown in the drawings and are not limiting upon the claims unless expressly recited therein.

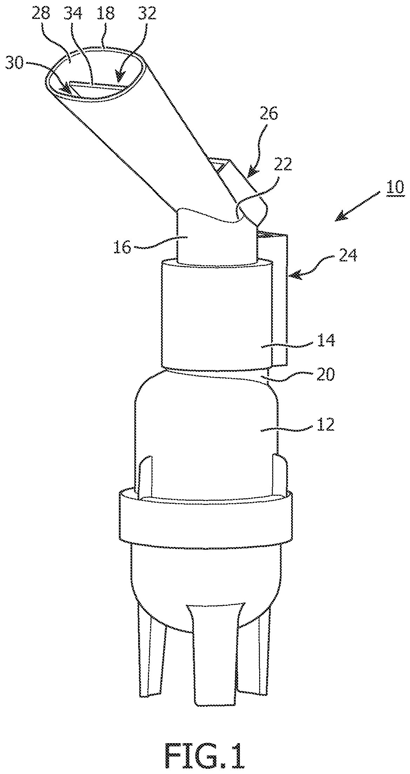

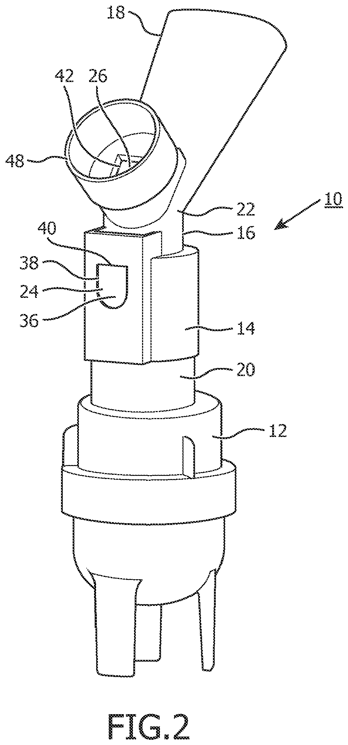

FIGS. 1 and 2 illustrate a mouthpiece 10 configured to be removably coupled with a nebulizer 12 to deliver nebulized medicament to the airway of a subject. Mouthpiece 10 is configured to connect to nebulizer 12 in a swappable manner with another mouthpiece (not shown) that is configured to operate in a continuous use mode. By contrast, mouthpiece 10 is configured to operate in a breath assist mode that reduces medicament waste caused, for example, by exhalation of the subject back into mouthpiece 10. This may be advantageous during the delivery some medicaments (*e.g*., steroids, antibiotics, bronchodialators, and/or other medicaments). Mouthpiece 10 is configured to maintain the double venturi effect that would be provided by the continuous use mouthpiece. In some embodiments, mouthpiece 10 includes one or more of a nebulizer interface 14, an outer housing 16, and a subject interface 18.

Nebulizer interface 14 is configured to releasable engage an outlet 20 of nebulizer 12. Outlet 20 of nebulizer 12 is the opening through which nebulizer 12 expels nebulized medicament for delivery to the subject. In order to engage outlet 20, nebulizer interface 14 may have a cross-sectional shape that corresponds to a cross-sectional shape of outlet 20. The releasable engagement between outlet 20 and nebulizer interface 14 may be accomplished via one or more of latch, snap, friction fit, threaded engagement, interconnecting protrusions and intrusions, and/or other releasably connecting mechanisms. The releasable engagement between outlet 20 and nebulizer interface 14 is such that mouthpiece 10 can be swapped out from outlet 20 for another mouthpiece, removed for cleaning, removed for storage, and/or released for other purposes, and then re-engaged for further use. Nebulizer interface 14 may be configured to releasably engage outlet 20 in the same, or substantially the same, manner in which a mouthpiece designed for continuous use would be engaged with outlet 20. This may enable nebulizer 12 to operate as both a continuous mode nebulizer (*e*.*g.*, with the other mouthpiece), a breath assisted nebulizer (*e.g*., with mouthpiece 10), as a breath-activated or dosimetric nebulizer, and/or according to other modes of operation. The engagement between nebulizer interface 14 and outlet 20 may establish a substantially air tight path for the nebulized medicament to pass through between nebulizer 12 and mouthpiece 10.

It will be appreciated that the illustration of mouthpiece 10 being connected directly to the body of nebulizer 12 in FIGS. 1 and 2 is not intended to be limiting. In some embodiments, a flexible conduit may extend from the body of nebulizer 12, and mouthpiece 10 may releasably connect with an outlet provided at the end of the flexible conduit. For the purposes of this disclosure, such an outlet provided at the end of the flexible conduit may be considered to be the outlet of nebulizer 12.

Outer housing 16 may be formed from a rigid material, and is configured to house form an outer surface of mouthpiece 10. Outer housing 16 extends from nebulizer interface 14 to subject interface 18. At a location between nebulizer interface 14 and subject interface 18, outer housing 16 defines a bend 22 in the general shape of nebulizer 12. By virtue of bend 22, the general shape of mouthpiece 10 may run in a general direction that is transverse to the general shape of mouthpiece 10 at or near nebulizer interface 14. This is so that mouthpiece 10 and/or nebulizer 12 can be conveniently grasped by the subject while receiving nebulized medicament from nebulizer 12. Outer housing 16 forms an air inlet 24 and an exhaust outlet 26. Air inlet 24 and exhaust outlet 26 are openings in outer housing 16 through which the exterior of mouthpiece 10 is communicated with the interior of mouthpiece 10.

Subject interface 18 is configured to communicate the interior of mouthpiece 10 with the airway of the subject. At subject interface 18, outer housing 16 forms an interface opening 28. At subject interface 18, the shape of outer housing 16 is configured to facilitate engagement by the mouth of the subject to facilitate inhalation of medicament by the subject through interface opening 28. For example, outer housing 16 may be shaped such that interface opening 28 has an elongated shape, with the axis of elongation corresponding to the orientation of the mouth of the subject.

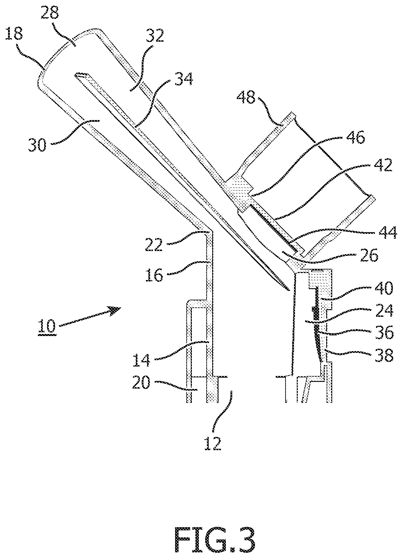

FIG. 3 illustrates a cross-sectional view of mouthpiece 10 and nebulizer 12. As can be seen in FIG. 3, within outer housing 16 mouthpiece 10 forms a medicament flow path 30 and an exhaust flow path 32. Medicament flow path 30 is configured to guide nebulized medicament from nebulizer interface 14 to subject interface 18 for inhalation by the subject. Medicament flow path 30 is formed by an inner surface of outer housing 16 and a dividing structure 34.

As can be seen in FIG. 3, air inlet 24 provides an opening in outer housing 16 that facilitates communication of ambient atmosphere with medicament flow path 30. This may enhance delivery of the nebulized medicament to the subject. For example, during inhalation, the negative pressure applied by the subject to medicament flow path 30 at subject interface 18 causes ambient air to be drawn into medicament flow path 30 through air inlet 24. The air drawn into medicament flow path 30 creates a flow of gas that carries nebulized medicament from nebulizer interface 14 to subject interface 18.

Mouthpiece 10 includes an inlet valve 36. Inlet valve 36 is a one-way valve configured to open ambient atmosphere with medicament flow path 30 during inhalation through air inlet 24 *(e.g.,* by virtue of the negative pressure applied by the subject), and to substantially close off communication between ambient atmosphere and medicament flow path 30 during exhalation (*e.g*., by virtue of a positive pressure applied by the subject during exhalation). This enables air to be drawn into medicament flow path 30 during inhalation through air inlet 24 to enhance the delivery of nebulized medicament as described above during inhalation, while preventing exhaled gas to flow back through medicament flow path 30 during exhalation to be expelled from mouthpiece 10 and/or nebulizer 12 *(e.g.,* through air inlet 24). In some embodiments, the only places at which gas enters and/or exits medicament flow path 30 may be nebulizer interface 14, subject interface 18, and air inlet 24.

As is shown in FIG. 3, in some embodiments, inlet valve 36 is disposed at or near air inlet 24. As was mentioned previously, inlet valve 36 is a one-way valve that permits gas to pass from outside mouthpiece 10 to the interior of mouthpiece 10, but substantially prevents gas from flowing out of the interior of mouthpiece 10. Inlet valve 36 may include an active valve, a passive valve, and/or other a combination of the two. By way of non-limiting example, inlet valve 36 may include one or more of a flapper valve, a plunger valve, and/or other valve types. In the embodiments illustrated in FIG. 3, inlet valve 36 is formed as a flapper valve by an elastic valve member 38 that covers air inlet 24 in its default position. Elastic valve member 38 is anchored to mouthpiece 10 at a first side 40. Responsive to a negative pressure being applied to medicament flow path 30 *(e.g.,* at inhalation), the force of the negative pressure on elastic valve member 38 causes elastic valve member 38 to elastically bend into medicament flow path 30, thereby uncovering air inlet 24 and permitting a flow of gas from outside mouthpiece 10 into medicament flow path 30 through air inlet 24. Responsive to the negative pressure stopping *(e.g.,* at the end of inhalation and/or during exhalation), elastic valve member 38 returns to its default position substantially sealing air inlet 24.

Air inlet 24 and/or inlet valve 36 may be disposed on and/or in mouthpiece 10 such that the flow of gas from air inlet 24 to subject interface 18 during inhalation passes across or near nebulizer interface 14. This may enhance the amount of medicament that is picked up in this flow of gas and carried from nebulizer interface 14 to subject interface 18 for inhalation by the subject.

Exhaust flow path 32 is configured to guide gas exhaled from the airway of the subject to exhaust outlet 26 so that such gas can be exhausted from mouthpiece 10. Exhaust flow path 32 is formed by an inner surface of outer housing 16 and dividing structure 34. Exhaust flow path 32 runs from subject interface 18 to exhaust outlet 26.

Exhaust outlet 26 provides an opening in outer housing 16 that facilitates communication of ambient atmosphere with exhaust flow path 32. This enables gas to flow from exhaust flow path 32 out of mouthpiece 10 through exhaust outlet 26. For example, during exhalation, the gas exhaled into subject interface 18 flows through interface opening 28, into exhaust flow path 32, and out of mouthpiece 10 through exhaust outlet 26.

Although each of medicament flow path 30 and exhaust flow path 32 communicated with interface opening 28, medicament flow path 30 and exhaust flow path 32 are formed separately from each other. In the embodiments illustrated by FIG. 3, this physical separation is provided by dividing structure 34. Further, the physical separation between medicament flow path 30 and exhaust flow path 32 is provided such that nebulized medicament, in order to reach exhaust flow path 32, must travel through the length of medicament flow path 30. This reduces the amount of medicament that moves into exhaust flow path 32 *(e.g.,* to be exhausted from mouthpiece 10).

During exhalation, inlet valve 36 closes, as was described above. This effectively reduces flow within medicament flow path 30 to zero or substantially zero, and forces exhaled has to pass into exhaust flow path 32 instead of medicament flow path 30. This traps some or all of the nebulized medicament within medicament flow path 30 at the beginning of exhalation in medicament flow path 30, and if additional medicament is nebulized during exhalation the newly nebulized medicament is trapped in nebulizer 12 and/or medicament flow path 30. The exhaust flow path 32, on the other hand, carries the exhaled gas of out mouthpiece 10 without bringing the exhaled gas into contact with most or all of the nebulized medicament in mouthpiece 10 and/or nebulizer 12. This isolation of the exhaled gas from medicament flow path 30 (and/or the nebulized medicament therein) may reduce medicament expelled from mouthpiece 10 during exhalation, which in turn may reduce medicament waste and/or contamination to the surrounding area by air-born medicament.

Mouthpiece 10 includes an exhaust valve 42. Exhaust valve 42 is a one-way valve configured to communicate exhaust flow path 32 with the exterior of mouthpiece 10 during exhalation through exhaust outlet 26, and to substantially close off communication between exhaust flow path 32 and the exterior of mouthpiece 10 during inhalation. This enables exhaled gas to be expelled from mouthpiece 10 through exhaust flow path 32 during exhalation, while preventing nebulized medicament gas to flow back through exhaust flow path 32 and out of mouthpiece 10 via exhaust outlet 26 during inhalation. In some embodiments, the only places at which gas enters and/or exits exhaust flow path 32 may be subject interface 18 and exhaust outlet 26.

As is shown in FIG. 3, in some embodiments, exhaust valve 42 is disposed at or near exhaust outlet 26. As was mentioned previously, exhaust valve 42 is a one-way valve that permits gas to pass out of mouthpiece 10, but substantially prevents gas from entering mouthpiece 10. Exhaust valve 42 may include an active valve, a passive valve, and/or other a combination of the two. By way of non-limiting example, exhaust valve 42 may include one or more of a flapper valve, a plunger valve, and/or other valve types. In the embodiments illustrated in FIG. 3, exhaust valve 42 is formed as a flapper valve by an elastic valve member 44 that covers exhaust outlet 26 in its default position. Elastic valve member 44 is anchored to mouthpiece 10 at a first side 46. Responsive to gas being exhaled into mouthpiece 10 through subject interface 18 *(e.g.,* at exhalation), the force of the exhaled gas on elastic valve member 44 causes elastic valve member 44 to elastically bend, thereby uncovering exhaust outlet 26 and permitting a flow of gas out of mouthpiece 10 through exhaust outlet 26. Responsive to the flow of exhaled gas stopping *(e.g.,* at the end of exhalation and/or during inhalation), elastic valve member 44 returns to its default position substantially sealing exhaust outlet 26.

Exhaust outlet 26 and/or exhaust valve 42 may be disposed at an end of exhaust flow path 32 opposite from subject interface 18 such that the flow of exhaled gas is carried away from subject interface 18 and directed away from the subject during exhalation. This may inhibit exhausted gas from being expressed from mouthpiece 10 back on the subject, which may be unpleasant for the subject.

In some embodiments, a filter port 48 is formed around exhaust outlet 26. In FIGS. 2 and 3 (not shown in FIG. 1), filter port 48 is formed as a ridge that protrudes out of outer housing 16 in an annular shape surrounding exhaust outlet 26. Filter port 48 is configured to releasably engage a filter through which exhaled gas exiting mouthpiece 10 through exhaust outlet 26 must pass before being released into ambient atmosphere.

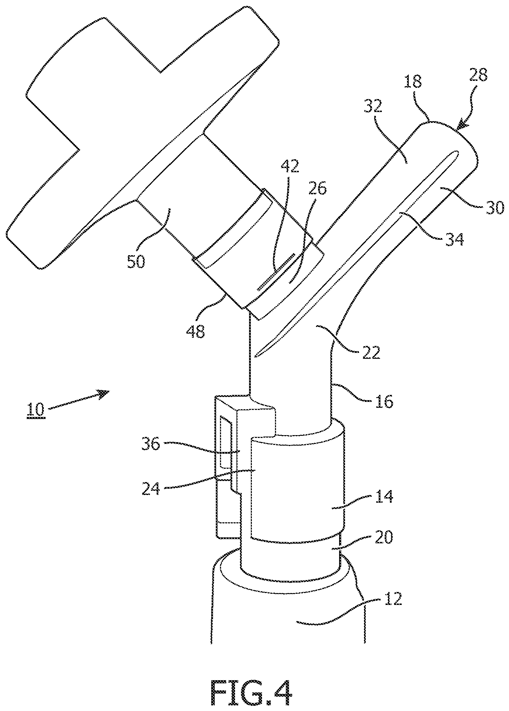

By way of illustration, FIG. 4 provides a view of mouthpiece 10 in which a filter 50 is engaged with filter port 48 to receive exhaled gas therethrough. Filter 50 may be configured to remove various agents from the exhaled gas before releasing the gas into ambient atmosphere. Such agents may include one or more of steroids, antibiotics, bronchodialators, and/or other substances. Filter port 48 may be configured to releasably engage filter 50 via one or more of latch, snap, friction fit, threaded engagement, interconnecting protrusions and intrusions, and/or other releasably connecting mechanisms.

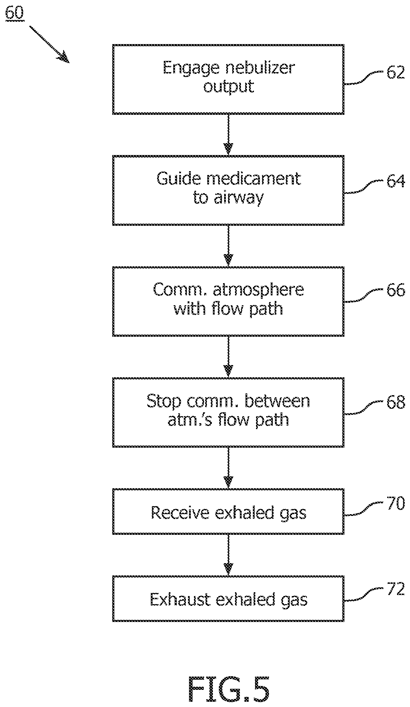

FIG. 5 illustrates a method 60 of delivering nebulized medicament to the airway of a subject. Method 60 may be implemented by a mouthpiece that is removable from a nebulizer. The operations of method 60 presented below are intended to be illustrative. In some examples , method 60 may be accomplished with one or more additional operations not described, and/or without one or more of the operations discussed. Additionally, the order in which the operations of method 60 are illustrated in FIG. 5 and described below is not intended to be limiting.

At an operation 62, an output of the nebulizer through which the nebulizer outputs nebulized medicament is releasably engaged. In some embodiments, operation 62 is performed by a nebulizer interface similar to or the same as nebulizer interface 14 (shown in FIGS. 1-3 and described herein).

At an operation 64, nebulized medicament is guided from the outlet of the nebulizer to the airway the subject through a medicament flow path during inhalation by the subject. In some embodiments, operation 64 is performed by a medicament flow path similar to or the same as medicament flow path 30 (shown in FIG. 3 and described herein).

At an operation 66, ambient atmosphere is placed in fluid communication with the medicament flow path during the inhalation by the subject. In some embodiments, operation 66 is performed by an air inlet and an inlet valve similar to or the same as air inlet 24 and inlet valve 36, respectively (as shown in FIGS. 2-4 and described herein).

At an operation 68, fluid communication between the medicament flow path and ambient atmosphere is stopped responsive to the end of the inhalation. In some embodiments, operation 68 is performed by the inlet valve.

At an operation 70, gas exhaled from the airway of the subject during an exhalation subsequent to the inhalation is received into an exhaust flow path. In some embodiments, operation 70 is performed by an exhaust flow path similar to or the same as exhaust flow path 32 (shown in FIG. 3 and described herein).

At an operation 72, the exhaust flow path is placed in fluid communication with an exterior of the mouthpiece in response to the exhalation so that exhaled gas can be exhausted from the mouthpiece through the exhaust flow path. In some embodiments, operation 72 is performed by an exhaust outlet and exhaust valve similar to or the same as exhaust outlet 26 and exhaust valve 42, respectively, (shown in FIGS. 2-4 and described herein).

In the claims, any reference signs placed between parentheses shall not be construed as limiting the claim. The word "comprising" or "including" does not exclude mere fact that certain elements are recited in mutually different dependent claims does not indicate that these elements cannot be used in combination.

Although the description provided above provides detail for the purpose of illustration based on what is currently considered to be the most practical and preferred embodiments, it is to be understood that such detail is solely for that purpose and that the disclosure is not limited to the expressly disclosed embodiments, but, on the contrary, is intended to cover modifications and equivalent arrangements that are within the scope of the appended claims. For example, it is to be understood that the present disclosure contemplates that, to the extent possible, one or more features of any embodiment can be combined with one or more features of any other embodiment.

## Claims

1. A mouthpiece (10) configured to be removably coupled with a nebulizer (12) to deliver nebulized medicament to the airway of a subject, the mouthpiece comprising:
a nebulizer interface (14) configured to releasably engage an outlet of a nebulizer through which the nebulizer outputs nebulized medicament;
a subject interface (18) suitable for engagement by the mouth of the subject and for communicating the interior of the mouthpiece with the airway of the subject;
a medicament flow path (30) configured to place the nebulizer interface in fluid communication with the subject interface to facilitate a flow of nebulized medicament from the nebulizer interface to the subject interface and into the airway of the subject through the subject interface;
an exhaust outlet (26);
an exhaust flow path (32) configured to place the subject interface in fluid communication with the exhaust outlet to facilitate a flow of exhaled gas from the subject interface to the exhaust outlet and out of the mouthpiece through the exhaust outlet;
an inlet valve (36) configured to communicate ambient air with the medicament flow path, wherein the inlet valve is a one-way valve that permits flow fromambient atmosphere into the medicament flow path; and
an exhaust valve (46) that controls gas flow within the exhaust flow path, wherein the outlet valve is a one-way valve that permits flow out of the exhaust outlet, **characterized in that** the mouthpiece (10) further comprises a dividing structure (34) that physically separates the medicament flow path from the exhaust flow path.

2. The mouthpiece of claim 1, wherein the inlet valve and the exhaust valve facilitate delivery of nebulized medicament to the airway of the subject in a breath enhanced mode.

3. The mouthpiece of claim 1, wherein the nebulizer interface is configured to releasably engage an outlet of the nebulizer that is configured to receive engagement of a continuous operation mouthpiece.

4. The mouthpiece of claim 1, configured to create a double venturi effect while deliverig medicament to the airway of the subject.

## Patentansprüche

1. Mundstück (10), das konfiguriert ist, um abnehmbar mit einem Zerstäuber (12) gekoppelt zu werden, um ein zerstäubtes Arzneimittel in die Atemwege eines Patienten zu verabreichen, wobei das Mundstück Folgendes umfasst:
eine Zerstäuberschnittstelle (14), die konfiguriert ist, um lösbar in einen Auslass eines Zerstäubers einzugreifen, durch den der Zerstäuber zerstäubtes Arzneimittel ausgibt;
eine Patientenschnittstelle (18), die für den Eingriff durch den Mund des Patienten und für die Herstellung einer kommunikativen Verbindung des Mundstückinneren mit den Atemwegen des Patienten geeignet ist;
einen Arzneimittelströmungspfad (30), der konfiguriert ist, um die Zerstäuberschnittstelle in Fluidkommunikation mit der Patientenschnittstelle zu bringen, um eine Strömung des zerstäubten Arzneimittels von der Zerstäuberschnittstelle zu der Patientenschnittstelle und durch die Patientenschnittstelle in die Atemwege des Patienten zu ermöglichen;
einen Abgasauslass (26);
einen Abgasströmungspfad (32), der konfiguriert ist, um die Patientenschnittstelle in Fluidkommunikation mit dem Abgasauslass zu bringen, um eine Strömung von ausgeatmetem Gas von der Patientenschnittstelle zum Abgasauslass und durch den Abgasauslass aus dem Mundstück heraus zu ermöglichen;
ein Einlassventil (36), das konfiguriert ist, um Umgebungsluft mit dem Arzneimittelströmungspfad in kommunikative Verbindung zu bringen, wobei das Einlassventil ein Einwegeventil ist, das Strömung von der Umgebungsatmosphäre in den Arzneimittelströmungspfad erlaubt; und
ein Auslassventil (46), das Gasströmung innerhalb des Abgasströmungspfads steuert, wobei das Auslassventil ein Einwegeventil ist, das Strömung aus dem Abgasauslass heraus erlaubt,
**dadurch gekennzeichnet, dass** das Mundstück (10) weiterhin eine Teilungsstruktur (34) umfasst, die den Arzneimittelströmungspfad von dem Abgasströmungspfad trennt.

2. Mundstück nach Anspruch 1, wobei das Einlassventil und das Auslassventil die Verabreichung von zerstäubtem Arzneimittel in die Atemwege des Patienten in einem atmungsunterstütztem Modus ermöglicht.

3. Mundstück nach Anspruch 1, wobei die Zerstäuberschnittstelle konfiguriert ist, um lösbar in einen Auslass des Zerstäubers einzugreifen, der konfiguriert ist, um einen Eingriff eines Dauerbetrieb-Mundstücks aufzunehmen.

4. Mundstück nach Anspruch 1, das konfiguriert ist, um einen Doppelventurieffekt zu erzeugen, während das Arzneimittel den Atemwegen des Patienten verabreicht wird.

## Revendications

1. Embout buccal (10) configuré pour être couplé de manière amovible à un nébuliseur (12) pour administrer un médicament nébulisé aux voies respiratoires d'un sujet, l'embout buccal comprenant :
une interface de nébuliseur (14) configurée pour engager de manière libérable une sortie d'un nébuliseur à travers laquelle le nébuliseur sort le médicament nébulisé ;
une interface de sujet (18) appropriée pour être engagée par la bouche du sujet et pour faire communiquer l'intérieur de l'embout buccal avec les voies respiratoires du sujet ;
un chemin d'écoulement de médicament (30) configuré pour placer l'interface de nébuliseur en communication de fluide avec l'interface de sujet afin de faciliter un écoulement de médicament nébulisé depuis l'interface de nébuliseur avec l'interface de sujet et dans les voies respiratoires du sujet par l'intermédiaire de l'interface de sujet ;
une sortie d'échappement (26) ;
un chemin d'écoulement d'échappement (32) configuré pour placer l'interface de sujet en communication de fluide avec la sortie d'échappement afin de faciliter un écoulement de gaz expiré depuis l'interface de sujet vers la sortie d'échappement et à l'extérieur de l'embout buccal par l'intermédiaire de la sortie d'échappement ;
un clapet d'entrée (36) configuré pour faire communiquer l'air ambiant avec le chemin d'écoulement de médicament, dans lequel le clapet d'entrée est un clapet unidirectionnel qui permet un écoulement depuis l'atmosphère ambiante dans le chemin d'écoulement de médicament ; et
un clapet d'échappement (46) qui commande l'écoulement de gaz dans le chemin d'écoulement d'échappement, dans lequel le clapet de sortie est un clapet unidirectionnel qui permet un écoulement à l'extérieur de la sortie d'échappement,
**caractérisé en ce que** l'embout buccal (10) comprend en outre une structure de division (34) qui sépare physiquement le chemin d'écoulement de médicament du chemin d'écoulement d'échappement.

2. Embout buccal selon la revendication 1, dans lequel le clapet d'entrée et le clapet d'échappement facilitent l'administration de médicament nébulisé aux voies respiratoires du sujet dans un mode respiration améliorée.

3. Embout buccal selon la revendication 1, dans lequel l'interface de nébuliseur est configurée pour engager de manière libérable une sortie du nébuliseur qui est configurée pour recevoir l'engagement d'un embout buccal à fonctionnement continu.

4. Embout buccal selon la revendication 1, configuré pour créer un double effet venturi tout en administrant un médicament aux voies respiratoires du sujet.
